# EUROPEAN PATENT APPLICATION

(11) **EP 2 820 949 A1**
(43) Date of publication of application: **07.01.2015**
(21) Application number: 13755837.5
(22) Date of filing: 14.02.2013
(51) Int. Cl.: A01M 1/20, A61L 9/14, B05B 17/06, B65D 83/00, B65D 83/76

(54) **LIQUID CONTAINER, ULTRASONIC ATOMIZATION DEVICE, AND ABSORPTION BODY**

(30) Priority: 29.02.2012 JP 2012044143
(71) Applicant: Sumitomo Chemical Company Limited, Tokyo 104-8260 (JP)
(72) Inventor: KAWANO, Hiroyuki, Takarazuka-shi Hyogo 665-8555 (JP); HARADA, Tetsuo, Takarazuka-shi Hyogo 665-8555 (JP); TAKAHATA, Daisuke, Ageo-shi Saitama 362-8561 (JP); UEDA, Kazuyuki, Ageo-shi Saitama 362-8561 (JP)
(74) Representative: Vossius & Partner
(86) International application number: PCT/JP2013/053567
(87) International publication number: WO 2013/129120

(57) **Abstract**

An ultrasonic atomizing device (1) includes (i) a liquid absorbent wick (22) which absorbs liquid from a solution container (20) and (ii) an absorber (23) which supplies, to a vibrating plate (32), the liquid absorbed by the liquid absorbent wick (22). The absorber (23) is configured to be provided to or removed from the ultrasonic atomizing device (1) together with the solution container (20) when the solution container (20) is provided to or removed from the ultrasonic atomizing device (1).

## Description

### Technical Field

The present invention relates to (i) a liquid container for use in an ultrasonic atomizing device which atomizes liquid such as water or a solution by ultrasonic vibration, (ii) an ultrasonic atomizing device, and (iii) an absorber.

### Background Art

An ultrasonic atomizing device has been known as means for atomizing, in an interior or exterior space, liquid such as a solution containing an active ingredient. The ultrasonic atomizing device includes (i) a piezoelectric element which generates ultrasonic vibration when supplied with electricity and (ii) a vibrating plate which has many micropores and is attached to the piezoelectric element. The ultrasonic atomizing device is configured so as to atomize liquid by (i) supplying the liquid to the micropores and (ii) causing ultrasonic vibration on the vibrating plate by vibration of the piezoelectric element.

A piezoelectric atomizing device of Patent Literature 1 includes a liquid absorbent wick, a liquid container and a piezoelectric atomizing section. The liquid absorbent wick is divided into a first section to pass the solution and a second section to pass the solution. The first section to pass the solution is provided to the liquid container, and the second section to pass the solution is provided to a body of the piezoelectric atomizing device.

According to a piezoelectric atomizing device of Patent Literature 2, a piezoelectric atomizing section and a liquid absorbent wick are attached to a liquid container. The piezoelectric atomizing section and the liquid absorbent wick, together with the liquid container, are removably contained in a body of the piezoelectric atomizing device.

### Citation List

### Patent Literatures

Patent Literature 1
   Japanese Patent Application Publication, Tokukaihei, No. 11-221505 A (Publication Date: August 17, 1999)
Patent Literature 2
   Japanese Patent Application Publication, Tokukai, No. 2000-51755 A (Publication Date: February 22, 2000)

### Summary of Invention

### Technical Problem

However, the piezoelectric atomizing devices of Patent Literatures 1 and 2 have the following problems.

Specifically, according to the piezoelectric atomizing device of Patent Literature 1, the second section to pass the solution is provided to a body of the piezoelectric atomizing device and is always in weak contact with or in contact with the vibrating plate. Therefore, when the solution container becomes empty and the second section to pass the solution dries, the micropores of the vibrating plate would be clogged with the fibers etc. of the second section to pass the solution. This may cause the amount of atomized solution to be unstable. In order to eliminate this cause, it is necessary to replace the second section to pass the solution or the vibrating plate. However, the replacement of the vibrating plate is costly. If a user carries out the replacement of the second section to pass the solution or the vibrating plate himself, the vibrating plate may be strongly pressed against the second section to pass the solution or may not be in sufficient contact with the second section to pass the solution, for example. These are problems for stable atomizing of the solution.

According to the piezoelectric atomizing device of Patent Literature 2, the piezoelectric atomizing section and the liquid absorbent wick are attached to the solution container. Therefore, when the solution container is replaced, the piezoelectric atomizing section is also to be replaced. This imposes a burden of high replacement costs on a user.

The present invention has been made in order to solve the above problems, and an object of the present invention is to provide a liquid container, an ultrasonic atomizing device and an absorber, each of which is capable of reducing a burden on a user.

### Solution to Problem

A liquid container in accordance with the present invention is a liquid container for being removably provided to an ultrasonic atomizing device, the ultrasonic atomizing device including a vibrating plate configured to be vibrated, by a piezoelectric element, for atomizing liquid, said liquid container including: a liquid absorbent wick for absorbing the liquid from the liquid container; and an absorber for supplying, to the vibrating plate, the liquid absorbed by the liquid absorbent wick, the absorber being configured to be provided to or removed from the ultrasonic atomizing device together with the liquid container when the liquid container is provided to or removed from the ultrasonic atomizing device.

According to the configuration, the ultrasonic atomizing device includes the vibrating plate, and the liquid container which is removably provided to the ultrasonic atomizing device has the liquid absorbent wick and the absorber. The absorber is, when the liquid container is provided to or removed from the ultrasonic atomizing device, provided to or removed from the ultrasonic atomizing device together with the liquid container.

That is, when the liquid container is removed from the ultrasonic atomizing device, the absorber is removed together with the liquid container and thus is not left in the ultrasonic atomizing device. That is, when the liquid container is to be replaced because the liquid container has become empty and the absorber has dried, the liquid container is replaced together with the absorber. This makes it possible to reduce the possibility of clogging micropores of the vibrating plate with fibers etc. derived from the absorber when the ultrasonic atomizing device is turned on again. As such, the liquid container in accordance with the present invention less causes the amount of atomized liquid to be unstable due to the clogging, and less causes a user to replace costly vibrating plates.

In addition, according to the liquid container in accordance with the present invention, since the piezoelectric element and the vibrating plate are included in the ultrasonic atomizing device, it is not necessary to replace the piezoelectric element and the vibrating plate when replacing the liquid container. This makes it possible to inexpensively replace the liquid container.

As has been described, the liquid container in accordance with the present invention makes it possible to reduce the burden on a user in terms of costs, and also possible to enhance atomization stability of the ultrasonic atomizing device because clogging of the micropores of the vibrating plate is suppressed.

### Advantageous Effects of Invention

As has been described, a liquid container in accordance with the present invention includes: a liquid absorbent wick for absorbing the liquid from the liquid container; and an absorber for supplying, to the vibrating plate, the liquid absorbed by the liquid absorbent wick, the absorber being configured to be provided to or removed from the ultrasonic atomizing device together with the liquid container when the liquid container is provided to or removed from the ultrasonic atomizing device.

Accordingly, it is possible to provide a liquid container capable of reducing a burden on a user.

### Brief Description of Drawings

Fig. 1

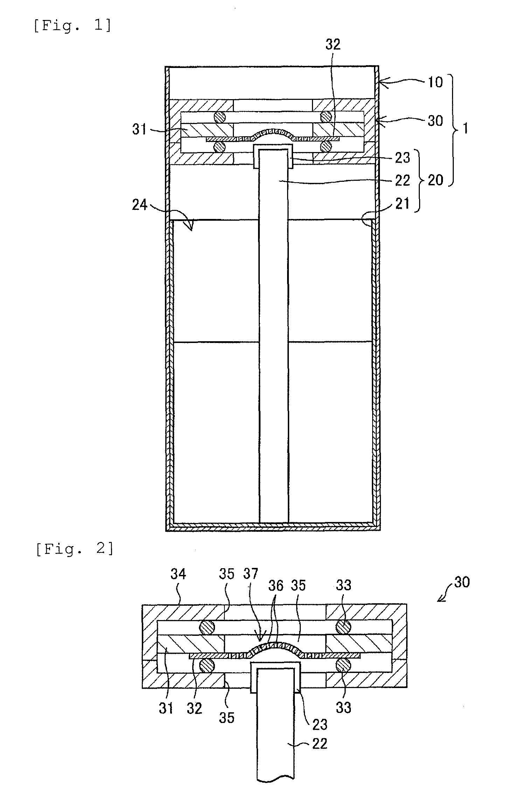

   Fig. 1 is a view schematically illustrating an ultrasonic atomizing device in accordance with the present embodiment.
Fig. 2
   Fig. 2 is an enlarged view of an atomization section of the ultrasonic atomizing device in accordance with the present embodiment.
Fig. 3

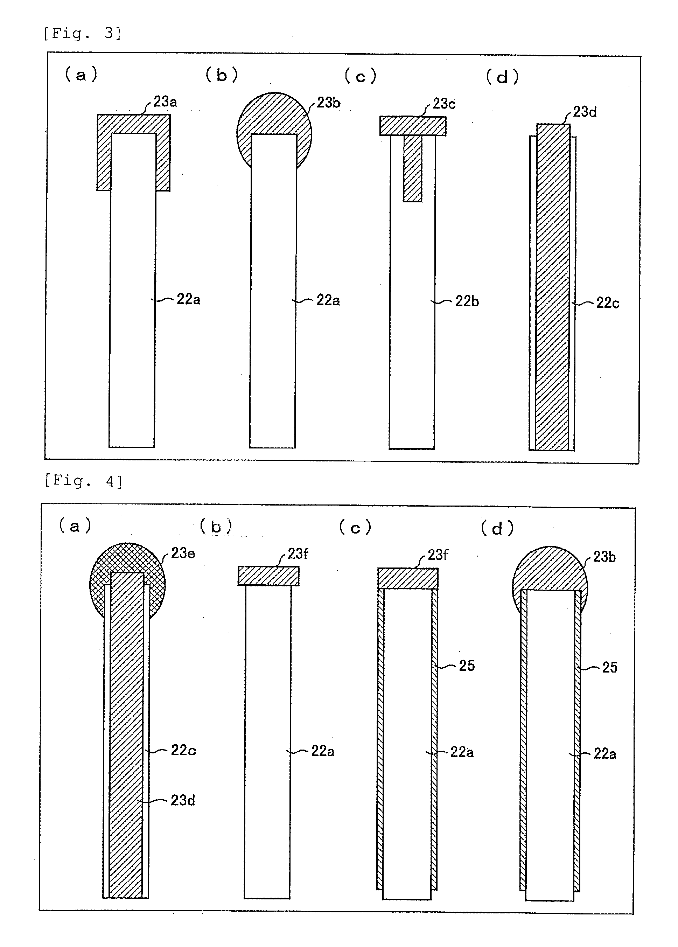

   Fig. 3 shows embodiments of an integrated structure in which a liquid absorbent wick is integral with an absorber. (a) of Fig. 3 shows a cap structure, (b) of Fig. 3 shows a cotton swab structure, (c) of Fig. 3 shows a fit-in structure, and (d) of Fig. 3 shows a double-wick structure.
Fig. 4
   Fig. 4 shows embodiments of an integrated structure in which a liquid absorbent wick is integral with an absorber. (a) of Fig. 4 shows a double-wick cotton swab structure, (b) of Fig. 4 shows a bonded structure, (c) of Fig. 4 shows a straw-shaped bonded structure, and (d) of Fig. 4 shows a straw-shaped cotton swab structure.

### Description of Embodiments

First, the following description discusses, with reference to Fig. 1 etc., an ultrasonic atomizing device 1, a solution container (liquid container) 20 and an absorber 23 in accordance with the present embodiment. Fig. 1 is a view schematically illustrating the ultrasonic atomizing device 1. Fig. 2 is an enlarged view of an atomization section 30 of the ultrasonic atomizing device 1.

### (Ultrasonic atomizing device 1)

The ultrasonic atomizing device 1 is a device for atomizing liquid such as water or a solution by ultrasonic vibration. The ultrasonic atomizing device 1 includes (i) a device body 10 which includes the atomization section 30 and (ii) a solution container 20 which is removably provided to the device body 10. The following description is based on the assumption that the liquid is water or a solution such as liquid of insecticide, pesticide or perfume.

### (Device body 10)

The device body 10 includes the atomization section 30, and is provided with the solution container 20 which is removable. The atomization section 30 includes (i) a piezoelectric element 31 which generates ultrasonic vibration when supplied with electricity, (ii) a vibrating plate 32 which atomizes a solution by vibration of the piezoelectric element 31, (iii) a couple of elastic rings 33 which are elastic annular members provided along a top surface of the piezoelectric element 31 and a bottom surface of the vibrating plate 32, respectively, and (iv) a casing 34 which holds the piezoelectric element 31 and the vibrating plate 32 by elastically sandwiching the piezoelectric element 31 and the vibrating plate 32 via the couple of elastic rings 33 (see Fig. 2).

The piezoelectric element 31 is constituted by a thin circular piezoelectric ceramic plate, which has an opening 35 at its center. The piezoelectric element 31 is polarized along its thickness direction, and generates ultrasonic vibration in a radial direction upon application of a high frequency voltage to electrodes (not illustrated) provided on both surfaces of the piezoelectric element 31. The piezoelectric element 31 is not limited provided that for example its thickness is 0.1 mm to 4.0 mm, its outer diameter is 6 mm to 60 mm, and its oscillatory frequency is 30 kHz to 500 kHz.

The vibrating plate 32 is constituted by a thin circular plate made of for example nickel. The vibrating plate 32 covers the opening 35 of the piezoelectric element 31, and, in Fig. 1, is joined (fastened) to a bottom surface of the piezoelectric element 31 so as to be concentric with the piezoelectric element 31. The thickness of the vibrating plate 32 is for example 0.02 mm to 2.0 mm, and the outer diameter of the vibrating plate 32 is for example 6 mm to 60 mm. The outer diameter of the vibrating plate 32 is selected as appropriate depending on the size of the piezoelectric element 31 so as to be larger than the inner diameter of the opening 35 of the piezoelectric element 31.

The vibrating plate 32 has, in its part that faces the opening 35 of the piezoelectric element 31, many micropores 36 passing through the vibrating plate 32 in a thickness direction. The diameter of each of the micropores 36 is preferably 3 µm to 150 µm.

The vibrating plate 32 has, at its center, a convex part 37 constituted by a curved surface from top to bottom. The convex part 37 is a dome-shaped part which protrudes upward (in a direction in which a solution is to be atomized). Since the center of the vibrating plate 32 is dome-shaped, it is possible to more easily atomize the solution extensively. The convex part 37 generates ultrasonic vibration in a vertical direction when the piezoelectric element 31 extends and contracts (vibrates) in the radial direction.

There is provided the couple of elastic rings 33. The couple of elastic rings 33 are in contact with the top surface of the piezoelectric element 31 and the bottom surface of the vibrating plate 32, respectively, so as to be concentric with the piezoelectric element 31 and the vibrating plate 32, respectively. Here, the couple of elastic rings 33 are in a state of elastic deformation between the casing 34 and the top surface of the piezoelectric element 31 and between the casing 34 and the bottom surface of the vibrating plate 32, respectively.

Each of the couple of elastic rings 33 is preferably an O-ring having a section diameter of 0.5 mm to 3 mm. Further, the hardness of the couple of elastic rings 33 is preferably 20 IRHD to 90 IRHD. Such a couple of elastic rings 33 makes it possible to hold the piezoelectric element 31 and the vibrating plate 32 with appropriate elasticity, and thus effectively prevent excessive vibration of the piezoelectric element 31 and the vibrating plate 32. Accordingly, it is possible to atomize a solution in a more stable manner.

It should be noted that an elastic ring 33 in contact with the top surface of the piezoelectric element 31 and an elastic ring 33 in contact with the bottom surface of the vibrating plate 32 are preferably the same in terms of mean diameter [(Inner diameter + Outer diameter) / 2], section diameter, and hardness etc. In particular, the couple of elastic rings 33 preferably have the same mean diameter.

The couple of elastic rings 33 are made from for example nitrile rubber, fluororubber, ethylene propylene rubber, silicone rubber, acrylic rubber, hydrogenated nitrile rubber, and/or the like.

Each of the couple of elastic rings 33 can be, instead of the O-ring, a ring that has for example an elliptic, rectangular, triangular or rhombic cross section. Alternatively, each of the couple of elastic rings 33 can be a ring that has for example a D-shaped, X-shaped or T-shaped cross section. Each of the couple of elastic rings 33 does not necessarily have to be a circumferentially continuous and complete ring, and therefore can have a slit in a circumferential direction or have several slits at regular intervals along the circumferential direction.

The convex part 37 of the vibrating plate 32 is not limited to a dome-shaped part whose top is constituted by a curved surface, and can have any shape such as a truncated cone-shaped part whose top is constituted by a flat surface.

The vibrating plate 32 is not limited to the convex vibrating plate which has the convex part 37 protruding in an atomization direction as described above as an example. The vibrating plate 32 can be a concave vibrating plate which has a concave part (i.e., a convex part 37 protruding in a direction opposite to the atomization direction). The vibrating plate 32 can be a flat vibrating plate which does not have any convex or concave part at its center.

Although the foregoing description discussed the vibrating plate 32 in the form of a thin circular plate which completely covers the opening 35 of the piezoelectric element 31, it is also possible to employ a vibrating plate in the form of a thin rectangular plate (i) which traverses the opening 35 of the piezoelectric element 31 and (ii) whose both ends are fastened to one surface of the piezoelectric element 31.

The atomization section 30 is not limited to the foregoing configuration, and can be constituted by a known piezoelectric atomization section. The atomization section 30 can be selected as appropriate.

### (Solution container 20)

The solution container 20 is constituted by a container body 21, a liquid absorbent wick 22 and an absorber 23, and is removably provided to the device body 10.

The container body 21 is constituted by for example a cylindrical container which has a bottom surface and has an opening 24 at the top. The container body 21 contains a solution. The container body 21 is made from for example glass or a synthetic resin.

The liquid absorbent wick 22 is for example made of nonwoven fabric and in columnar shape having a diameter of 2 mm to 6 mm. A lower portion of the liquid absorbent wick 22 is immersed in the solution contained in the container body 21. This makes it possible to supply the solution to an upper portion of the liquid absorbent wick 22 by capillary action. The absorber 23 is provided to the upper portion of the liquid absorbent wick 22.

The shape of the liquid absorbent wick 22 is not limited to a circular column, and can be a square column. The shape of the liquid absorbent wick 22 can be any shape. Furthermore, the thickness of the liquid absorbent wick 22 is not limited provided that the liquid absorbent wick 22 can pass through the opening 35 of the piezoelectric element 31.

The absorber 23 is provided to the upper portion of the liquid absorbent wick 22 so as to be integral with the liquid absorbent wick 22. That is, when the solution container 20 is provided to or removed from the ultrasonic atomizing device 1, the absorber 23 is also provided to or removed from the ultrasonic atomizing device 1 together with the solution container 20. The absorber 23 lies near or is in contact with the convex part 37 of the vibrating plate 32, and supplies, to the convex part 37, the solution absorbed by the liquid absorbent wick 22. This makes it possible to atomize the solution from the vibrating plate 32, and also possible to keep the stability of atomization amount. This will be described later in detail in Effect Confirmation Test.

The integrated structure in which the liquid absorbent wick 22 is integral with the absorber 23 can be embodied in various manners. Some of them will be described later with reference to Figs. 3 and 4. In the following description, the integrated structure in which the liquid absorbent wick 22 is integral with the absorber 23 may be referred to as a "double-wick integrated structure".

In the present embodiment, the term "integral" means (i) members constitute a single member, (ii) members are assembled together, or (iii) the like.

The absorbent wick 22 and/or the absorber 23 are/is fixed to the container body 21, and removably attached to the solution container 20 (or the container body 21).

The liquid absorbent wick 22 and the absorber 23 are each preferably made of, for example, a porous material having continuous holes, an open-cell resin article, or an aggregation of resin fibers. Specific examples of materials from which the liquid absorbent wick 22 and the absorber 23 are made include, but not limited to: open-cell resin articles made of polyurethane, polyethylene, polyethylene terephthalate, polyvinyl formal and polystyrene etc.; porous materials obtained by sintering of fine resin particles made mainly of polyethylene, polypropylene, and nylon etc.; porous materials made of polyethylene fluoride etc.; aggregations of resin fibers, such as felt made of polyester, polypropylene, nylon, acrylic, rayon, wool etc. and nonwoven fabric made of polyolefin fibers, polyester fibers, nylon fibers, rayon fibers, acrylic fibers, vinylon fibers, polychlal fibers, aramid fibers etc.; and porous sintered inorganic materials obtained by sintering of mainly inorganic powder such as ceramics. The specific examples of the materials further include the above materials treated with a surfactant. The liquid absorbent wick 22 and the absorber 23 can be made of the same material or of different materials.

How to provide the solution container 20 to the device body 10 is not particularly limited, provided that (i) the solution container 20 is removably provided to the device body 10 and, (ii) while the device body 10 is provided with the solution container 20, the absorber 23 is near or in contact with the convex part 37 of the vibrating plate 32. For example, the solution container 20 can be provided to the device body 10 by (i) being slidingly fitted into the device body 10 by being slid laterally or (ii) being rotatingly fitted into the device body 10 by being rotated laterally with a slight rotational angle.

### (Integrated structure in which liquid absorbent wick 22 is integral with absorber 23)

The following description discusses, with reference to Figs. 3 and 4, embodiments of an integrated structure in which the liquid absorbent wick 22 is integral with the absorber 23. Figs. 3 and 4 show embodiments of the integrated structure in which the liquid absorbent wick 22 is integral with the absorber 23. (a) of Fig. 3 shows a cap structure, (b) of Fig. 3 shows a cotton swab structure, (c) of Fig. 3 shows a fit-in structure, and (d) of Fig. 3 shows a double-wick structure. (a) of Fig. 4 shows a double-wick cotton swab structure, (b) of Fig. 4 shows a bonded structure, (c) of Fig. 4 shows a straw-shaped bonded structure, and (d) of Fig. 4 shows a straw-shaped cotton swab structure.

It should be noted that Figs. 3 and 4 show basic shapes of the embodiments, and therefore length, depth, width, and relative sizes and relative positions of the liquid absorbent wick 22 and the absorber 23 can be altered as appropriate. Since the vibrating plate 32 is provided for the upper part and the solution container 20 is provided for the lower part of each of Figs. 3 and 4, the absorber 23 is near or in contact with the vibrating plate 32 (not illustrated) which is provided for the upper part of each of Figs. 3 and 4.

First, the following description discusses the cap structure shown in (a) of Fig. 3. The cap structure is a structure in which a top end of a liquid absorbent wick 22a is capped with an absorber 23a, which is in a U shape (concave shape), so as to be fitted in a concave part of the absorber 23a. In this way, the absorber 23a is provided so as to cap the top end of the liquid absorbent wick 22a and is integral with the liquid absorbent wick 22a. The absorber 23 shown in Fig. 2 has the cap structure. According to this structure, liquid-holding capacity of the absorber 23a facilitates stable supply of a solution to the vibrating plate 32 (not illustrated) provided at the top of (a) of Fig. 3.

Next, the following description discusses the cotton swab structure shown in (b) of Fig. 3. As illustrated in (b) of Fig. 3, the cotton swab structure is a structure in which an absorber 23b is integral with a liquid absorbent wick 22a, so that a shape defined by outer shapes of the liquid absorbent wick 22a and the absorber 23b resembles a cotton swab. According to this structure, the absorber 23b (i) is capable of supplying a solution stably to the vibrating plate 32 provided for the upper part of (b) of Fig. 3, because of its liquid-holding capacity and (ii) has a shape that corresponds to a convex shape of the vibrating plate 32.

The following description discusses the fit-in structure shown in (c) of Fig. 3. As illustrated in (c) of Fig. 3, the fit-in structure is a structure in which an absorber 23c has a T-shaped cross section and a bar-shaped part of the T shape is inserted in a liquid absorbent wick 22b. According to this structure, it is possible to hold the absorber 23c on the liquid absorbent wick 22b in a structurally stable manner.

The following description discusses the double-wick structure shown in (d) of Fig. 3. The double-wick structure is a structure in which an absorber 23d in the shape of a column is inserted and fitted in a liquid absorbent wick 22c along an entire axis of the liquid absorbent wick 22c. That is, the absorber 23d is inserted and fitted in the liquid absorbent wick 22c along the entire length of the liquid absorbent wick 22c, and one end of the absorber 23d is immersed in a solution in the container body 21. Therefore, according to the double-wick structure, the liquid absorbent wick 22c and the absorber 23d absorb the solution (liquid absorption) from the container body 21. Further, the absorber 23d plays a role of feeding the vibrating plate 32 with the solution absorbed by the liquid absorbent wick 22c, because the other end of the absorber 23d is near or in contact with the vibrating plate 32. This structure has such an advantage that, even in a case where the absorber 23d absorbs a solution slowly (e.g., in a case where the absorber 23d has a low porosity), it is possible to supply the solution stably to the vibrating plate 32 (not illustrated) by using the liquid absorbent wick 22c which absorbs the solution fast (e.g., the liquid absorbent wick 22c which has a high porosity).

As used herein the porosity is calculated from the following equation: Porosity = {1 - (Weight of liquid absorbent wick or Weight of absorber) / [(Volume of liquid absorbent wick or Volume of absorber) × (Density of material for liquid absorbent wick or Density of material for absorber)]} x 100. The same applies to the following Examples.

The following description discusses the double-wick cotton swab structure shown in (a) of Fig. 4. As illustrated in (a) of Fig. 4, the double-wick cotton swab structure is a combination of the cotton swab structure shown in (b) of Fig. 3 and the double-wick structure shown in (d) of Fig. 3. The double-wick cotton swab structure includes a liquid absorbent wick 22c, an absorber 23d and an absorber 23e. The absorber 23d and the absorber 23e can be made of the same material or different materials. This structure has such an advantage that, even in a case where the absorber 23d absorbs a solution slowly (e.g., in a case where the absorber 23d has a low porosity), it is possible to supply the solution stably to the vibrating plate 32 by using the liquid absorbent wick 22c which absorbs the solution fast (e.g., the liquid absorbent wick 22c which has a high porosity). Furthermore, the absorber 23e is (i) capable of supplying the solution stably to the vibrating plate 32, because of its liquid-holding capacity and (ii) has a shape that corresponds to the convex shape of the vibrating plate 32.

The following description discusses the bonded structure shown in (b) of Fig. 4. The bonded structure is a structure in which an absorber 23f is bonded to a liquid absorbent wick 22a with use of an adhesion member such as an adhesive agent. The adhesion member preferably has properties that do not interrupt the supply of a solution from the liquid absorbent wick 22a to the absorber 23f. The adhesion member can be applied to the entire surfaces of the liquid absorbent wick 22a and the absorber 23f which surfaces are to be in contact with each other, or can be applied to only part of the surfaces. If the adhesion member is applied to only part of the surfaces, it is possible to reduce raw material costs.

The following description discusses the straw-shaped bonded structure shown in (c) of Fig. 4. The straw-shaped bonded structure is a structure in which a liquid absorbent wick 22a is inserted and fitted in a straw-like tube 25. An absorber 23f is integral with one end (for the vibrating plate side) of the liquid absorbent wick 22a which is inserted and fitted in the straw-like tube 25. The straw-like tube 25 is made of a material that does not absorb solutions. According to this structure, since a solution can be absorbed only by an end portion placed in the lower part of (c) of Fig. 4, it is possible to (i) prevent the speed of liquid absorption from being affected by the height from the bottom surface of the container to a surface of the solution and (ii) prevent spontaneous evaporation from the liquid absorbent wick 22a.

The following description discusses the straw-shaped cotton swab structure shown in (d) of Fig. 4. As illustrated in (d) of Fig. 4, the straw-shaped cotton swab structure is the same as the straw-shaped bonded structure shown in (c) of Fig. 4 except that the absorber 23b shown in (b) of Fig. 3 is used instead of the absorber 23f. The straw-shaped cotton swab structure includes a liquid absorbent wick 22a, the absorber 23b and a tube 25. According to the straw-shaped cotton swab structure, since the absorber 23b is capable of absorbing a solution only via one end portion of the liquid absorbent wick 22a, it is possible to prevent the speed of liquid absorption from being affected by the height from the bottom surface of the container to the surface of the solution. Furthermore, the tube 25 prevents spontaneous evaporation from the liquid absorbent wick 22a. Moreover, according to the straw-shaped cotton swab structure, the absorber 23b is (i) capable of supplying the solution stably to the vibrating plate 32, because of its liquid-holding capacity and (ii) has a shape that corresponds to the convex shape of the vibrating plate 32.

The foregoing descriptions discussed various embodiments with reference to Figs. 3 and 4. As has been described, the absorber 23 which can have various shapes and structures is provided so as to be integral with the liquid absorbent wick 22.

As illustrated in Figs. 3 and 4, a surface of the absorber 23, which surface is to be in contact with the vibrating plate 32, can have various shapes such as a convex, concave or flat shape. Note however that the surface of the absorber 23, which surface is to be in contact with the vibrating plate 32, preferably has a shape that corresponds to a surface of the vibrating plate 32 which surface is to be in contact with the absorber 23. That is, it is preferable that, in a case where the vibrating plate 32 has a concave, convex or flat shaped surface opposite to a surface from which a solution is to be atomized, the surface of the absorber 23 which surface is to be in contact with the vibrating plate 32 has a convex, concave or flat shape, accordingly.

Such an absorber 23 makes it possible to keep a good contact between the vibrating plate 32 and the absorber 23. This reduces or eliminates the factors which would affect the atomization stability of the solution, which factors are for example excessive or insufficient contact between the vibrating plate 32 and the absorber 23. As has been described, the liquid absorbent wick 22 and/or the absorber 23 can have various integrated structures depending on the shape and/or characteristics of the vibrating plate 32. This makes it possible to achieve optimum atomizing of a solution.

The liquid absorbent wick 22 and/or the absorber 23 can be provided so as to be (i) fixed to the container body 21 but (ii) detachable from the solution container 20 (or the container body 21). This provides such an advantage that, for example in a case where the liquid absorbent wick 22 and/or the absorber 23 have/has a failure but there is still some solution left in the solution container 20, it is possible to replace only the liquid absorbent wick 22 and/or the absorber 23 to thereby allow the ultrasonic atomizing device 1 to operate without losing atomization stability. This makes it possible to provide added value for a user, such as reduced costs for parts (members) replacement and effective use of solutions.

### (Effect Confirmation Test)

The following description discusses the present invention in more detail with Examples. Note, however, that these Examples do not imply any limitation on the present invention.

### (Production of ultrasonic atomizing device)

An ultrasonic atomizing device having the following specifications was produced.
(1) Piezoelectric element 31: Piezoelectric ceramics whose outer diameter is 15 mm, inner diameter is 5 mm and thickness is 0.4 mm
(2) Vibrating plate 32: Convex vibrating plate
(3) Applied voltage: 30 Vp-p
(4) Frequency of piezoelectric element 31 (ultrasonic exciter): 110 kHz

### (Production of wick having double-wick integrated structure)

Wicks each having a double-wick integrated structure, which have the following specifications, were produced.

### (Wick A having double-wick integrated structure)

A wick A having a double-wick integrated structure used in this effect confirmation test corresponds to the cap structure shown in (a) of Fig. 3.
(1) Liquid absorbent wick 22: Aggregate of polypropylene resin fibers and polyethylene resin fibers, whose inner diameter is 4.5 mm
(2) Absorber 23: Aggregate of wood pulp and synthetic fibers (Product name: AY-80 (produced by OJI KINOCLOTH CO., LTD.))
(3) Integrated structure: Liquid absorbent wick 22 is capped with absorber 23, and liquid absorbent wick 22 and absorber 23 are held by seal tube

### (Wick B having a double-wick integrated structure)

A wick B having a double-wick integrated structure used in this effect confirmation test corresponds to the cap structure shown in (b) of Fig. 3.
(1) Liquid absorbent wick 22: Aggregate of polypropylene resin fibers and polyethylene resin fibers, whose inner diameter is 3.5 mm
(2) Absorber 23: Aggregate of wood pulp and synthetic fibers
(3) Integrated structure: Absorber 23 is placed around and held to liquid absorbent wick 22

### (Wick C having a double-wick integrated structure)

A wick C having a double-wick integrated structure used in this effect confirmation test corresponds to the cap structure shown in (d) of Fig. 4.
(1) Liquid absorbent wick 22: Aggregate of polypropylene resin fibers and polyethylene resin fibers, whose inner diameter is 3.5 mm
(2) Tube 25: Tube made of polypropylene, whose inner diameter is 3.5 mm and outer diameter is 4.5 mm (2) Absorber 23: Aggregate of wood pulp and synthetic fibers (Product name: BEMCOT M-3II (produced by Asahi Kasei Corporation))
(3) Integrated structure: Absorbent wick 22 is inserted into tube 25, and absorber 23 is placed around and held to tube 25

### (Example 1)

The wick A having the double-wick integrated structure was held, with an inner plug, to a container body 21 filled with a solution (EXXSOL D110 (produced by Exxon Mobil Corporation)). The solution was atomized for one (1) second with use of an ultrasonic atomizing device 1. After the solution was atomized 10 times, the amount of atomized solution per atomizing was calculated from a difference between weights before and after the atomizing. This test was conducted 4 times, and relative standard deviation was calculated from the results of 4 tests. As a result, the amount per atomizing was 13.0 mg and the relative standard deviation was 0.6%.

### (Example 2)

The wick B having the double-wick integrated structure was held, with an inner plug, to a container body 21 filled with a solution (EXXSOL D110 (produced by Exxon Mobil Corporation)). The solution was atomized for one (1) second with use of an ultrasonic atomizing device 1. After the solution was atomized 10 times, the amount of atomized solution per atomizing was calculated from a difference between weights before and after the atomizing. This test was conducted 4 times, and relative standard deviation was calculated from the results of 4 tests. As a result, the amount per atomizing was 11.9 mg and the relative standard deviation was 1.0%.

### (Example 3)

The wick C having the double-wick integrated structure was held, with an inner plug, to a container body 21 filled with a solution (EXXSOL D110 (produced by Exxon Mobil Corporation)). The solution was atomized for one (1) second with use of an ultrasonic atomizing device 1. After the solution was atomized 10 times, the amount of atomized solution per atomizing was calculated from a difference between weights before and after the atomizing. This test was conducted 4 times, and relative standard deviation was calculated from the results of 4 tests. As a result, the amount per atomizing was 9.3 mg and the relative standard deviation was 3.1%.

### (Comparative Example 1)

A liquid absorbent wick 22 was held, with an inner plug, to a container body 21 filled with a solution (EXXSOL D110). The solution was atomized for one (1) second with use of an ultrasonic atomizing device 1. After the solution was atomized 10 times, the amount of atomized solution per atomizing was calculated from a difference between weights before and after the atomizing. This test was conducted 4 times, and relative standard deviation was calculated from the results of 4 tests. As a result, the amount per atomizing was 8.7 mg and the relative standard deviation was 5.0%.

### (Comparative Example 2)

A tube 25 in which a liquid absorbent wick 22 had been inserted was held, with an inner plug, to a container body 21 filled with a solution (EXXSOL D110). The solution was atomized for one (1) second with use of an ultrasonic atomizing device. After the solution was atomized 10 times, the amount of atomized solution per atomizing was calculated from a difference between weights before and after the atomizing. This test was conducted 4 times, and relative standard deviation was calculated from the results of 4 tests. As a result, the amount per atomizing was 5.1 mg and the relative standard deviation was 12.4%.

A comparison between the results of Examples 1 and 2 and the result of Comparative Example 1 showed that, with use of the wick having the double-wick integrated structure (Examples 1 and 2), the amount of atomized solution is large and the atomization is more stable as compared to a structure which only includes the liquid absorbent wick 22 and does not include the absorber 23 (Comparative Example 1).

Further, a comparison between the result of Example 3 and the result of Comparative Example 2 showed that, with use of the wick having the double-wick integrated structure (Example 3), the amount of atomized solution is large and the atomization is more stable as compared to a structure which only includes the liquid absorbent wick 22 inserted in the tube 25 and does not include the absorber 23 (Comparative Example 2).

### (Comparative Example 3)

A liquid absorbent wick 22 was held, with an inner plug, to a container body 21 filled with a solution (EXXSOL D110), and an absorber 23 was provided for a vibrating plate 32 side (such a structure is hereinafter referred to as a wick having a double-wick separated structure). The solution was atomized for one (1) second with use of an ultrasonic atomizing device. After the solution was atomized 10 times, the amount of atomized solution per atomizing was calculated from a difference between weights before and after the atomizing. This test was conducted 4 times, and relative standard deviation was calculated from the results of 4 tests. As a result, the amount per atomizing was 13.4 mg and the relative standard deviation was 0.7%.

A comparison between the results of Example 1 and Comparative Example 3 showed that the wick having the double-wick integrated structure and the wick having the double-wick separated structure are not so different in terms of the amount of atomized solution and the atomization stability.

### (Example 4)

The wick A having the double-wick integrated structure was held, with an inner plug, to a container body 21 filled with a solution (EXXSOL D110 (produced by Exxon Mobil Corporation)). The solution was atomized for one (1) second with use of an ultrasonic atomizing device 1. After the solution was atomized 10 times, the amount of atomized solution per atomizing was calculated from a difference between weights before and after the atomizing. Furthermore, the container body 21 in which the wick A having the double-wick integrated structure had been held with the inner plug was removed from the ultrasonic atomizing device 1, and allowed to stand for 7 days. After 7 days, the container body 21 in which the wick A having the double-wick integrated structure had been held with the inner plug was again provided to the ultrasonic atomizing device 1, and the solution was atomized for one (1) second. After the solution was atomized 10 times, the amount of atomized solution per atomizing was calculated from a difference between weights before and after the atomizing. As a result, the amount per atomizing at first was 12.9 mg, and the amount per atomizing after 7 days was 13.1 mg.

### (Comparative Example 4)

A liquid absorbent wick 22 was held, with an inner plug, to a container body 21 filled with a solution (EXXSOL D110), and an absorber 23 was provided for a vibrating plate 32 side. The solution was atomized for one (1) second with use of an ultrasonic atomizing device 1. After the solution was atomized 10 times, the amount of atomized solution per atomizing was calculated from a difference between weights before and after the atomizing. Furthermore, the container body 21 in which the liquid absorbent wick 22 had been held with the inner plug was removed from the ultrasonic atomizing device 1, and allowed to stand for 7 days. Meanwhile, the absorber 23 was left on the vibrating plate 32. After 7 days, the container body 21 in which the liquid absorbent wick 22 had been held with the inner plug was again provided to the ultrasonic atomizing device 1, and the solution was atomized for one (1) second. After the solution was atomized 10 times, the amount of atomized solution per atomizing was calculated from a difference between weights before and after the atomizing. As a result, the amount per atomizing at first was 13.2 mg, and the amount per atomizing after 7 days was 9.8 mg.

A comparison between Example 4 and Comparative Example 4 showed that (i) in a case of the wick having the double-wick separated structure, the amount of atomized solution decreases as the absorber 23 dries, but (ii) in a case of the wick having the double-wick integrated structure, the amount of atomized solution does not decrease even when the absorber 23 dries.

The above results showed that the wick having the double-wick integrated structure of the present embodiment (i) compares favorably with the wick having the double-wick separated structure in terms of the amount of atomized solution and the atomization stability and (ii) is more excellent than the structures of Comparative Examples 1 and 2 (the structure which only uses a liquid absorbent wick) in terms of the amount of atomized solution and the atomization stability. The results showed that these advantages are achieved by employing a structure of the absorber which is integral with the liquid absorbent wick.

The results also showed that, with use of the wick having the double-wick integrated structure of the present examples, it is possible to prevent the micropores of the vibrating plate from being clogged with fibers etc. of a dried absorber, and thus possible to prevent the amount of atomized solution from being unstable.

The foregoing descriptions discussed various configurations of a solution container, an ultrasonic atomizing device and an absorber in accordance with the present embodiment. These configurations serve as examples of the present embodiment, and it is needless to say that configurations described in the present embodiment can be combined.

An ultrasonic atomizing device in accordance with the present embodiment can be one which has the following configuration. That is, the liquid container in accordance with the present invention can be configured, such that the liquid absorbent wick and the absorber are detachably attached to the liquid container.

According to the configuration, for example in a case where the liquid absorbent wick and/or the absorber have/has a failure and there is some solution remaining in the liquid container, it is only necessary to replace only the liquid absorbent wick and the absorber. This makes it possible to provide added value for a user, such as reduced replacement cost and effective use of solutions.

An ultrasonic atomizing device in accordance with the present invention can include: the piezoelectric element; the vibrating plate; and the liquid container.

According to the configuration, when replacing the liquid container which has become empty, it is not necessary to also replace the piezoelectric element and the vibrating plate of the ultrasonic atomizing device, and thus is possible to keep using those piezoelectric element and vibrating plate. Furthermore, by using the above liquid container, micropores of the vibrating plate are less clogged with fibers etc. derived from the absorber.

Accordingly, the ultrasonic atomizing device in accordance with the present invention brings about the following effects: a user is not forced to replace the vibrating plate; and the amount of atomized liquid becomes stable.

An absorber in accordance with the present invention can be configured, such that its surface to be in contact with the vibrating plate has a shape that corresponds to a surface, of the vibrating plate, which is to be in contact with the absorber.

The absorber in accordance with the present invention can be configured, such that its surface to be in contact with the vibrating plate has a convex, concave or flat shape.

The shape of a surface, opposite to a surface from which the liquid is to be atomized, of the vibrating plate for use in the ultrasonic atomizing device can have various shapes such as a concave, convex or flat shape.

In this regard, the absorber in accordance with the present invention can be configured, such that its surface to be in contact with the vibrating plate has a shape that corresponds to the surface, of the vibrating plate, which is to be in contact with the absorber. That is, for example in a case where the vibrating plate has a concave, convex or flat shaped surface opposite to a surface from which a solution is to be atomized, the surface of the absorber in accordance with the present invention, which surface is to be in contact with the vibrating plate, can have a convex, concave or flat shape, accordingly.

Such an absorber in accordance with the present invention makes it possible to keep a good contact between the vibrating plate and the absorber. This eliminates the factors which would affect atomization stability of the solution, which factors are attributed to excessive or insufficient contact between the vibrating plate and the absorber.

The present invention is not limited to the descriptions of the respective embodiments, but may be altered within the scope of the claims. That is, an embodiment derived from a combination of technical means altered as appropriate within the scope of the claims is encompassed in the technical scope of the invention.

### Industrial Applicability

The present invention relates to a liquid container which is capable of reducing a burden on a user. In particular, the present invention is suitably applicable to an ultrasonic atomizing device.

### Reference Signs List

- 1: Ultrasonic atomizing device
- 10: Device body
- 20: Solution container (liquid container)
- 21: Container body
- 22: Liquid absorbent wick
- 23: Absorber
- 30: Atomization section
- 31: Piezoelectric element
- 32: Vibrating plate
- 33: Elastic ring
- 34: Casing
- 35: Opening
- 36: Micropores
- 37: Convex part

## Claims

1. A liquid container for being removably provided to an ultrasonic atomizing device, the ultrasonic atomizing device including a vibrating plate configured to be vibrated, by a piezoelectric element, for atomizing liquid, said liquid container comprising:
a liquid absorbent wick for absorbing the liquid from the liquid container; and
an absorber for supplying, to the vibrating plate, the liquid absorbed by the liquid absorbent wick,
the absorber being configured to be provided to or removed from the ultrasonic atomizing device together with the liquid container when the liquid container is provided to or removed from the ultrasonic atomizing device.

2. The liquid container according to claim 1, wherein the liquid absorbent wick and the absorber are detachably attached to the liquid container.

3. An ultrasonic atomizing device, comprising:
a liquid container recited in claim 1 or 2;
the piezoelectric element; and
the vibrating plate.

4. An absorber for use in a liquid container recited in claim 1 or 2, whose surface to be in contact with the vibrating plate has a shape that corresponds to a surface, of the vibrating plate, which is to be in contact with the absorber.

5. The absorber according to claim 4, whose surface to be in contact with the vibrating plate has a convex, concave or flat shape.
